(19) Europäisches Patentamt
European Patent Office
Office européen des brevets

(11) Veröffentlichungsnummer: **0 000 016**
**B1**

(12) **EUROPÄISCHE PATENTSCHRIFT**

(21) Anmeldenummer: **78100028.6**

(22) Anmeldetag: **01.06.78**

(51) Int. Cl.³: **C 07 C 127/22,**
C 08 G 18/80,
C 08 G 18/78

(54) **Verfahren zur Herstellung von Allophanatgruppen aufweisenden Polyisocyanaten und ihre Verwendung als Aufbaukomponente für Polyurethankunststoffe**

(30) Priorität: **04.06.77 DE 2725318**

(43) Veröffentlichungstag der Anmeldung:
**20.12.78 Patentblatt 78/01**

(45) Bekanntmachung des Hinweises auf die Patenterteilung:
**23.07.80 Patentblatt 80/15**

(84) Benannte Vertragsstaaten:
**BE DE FR GB NL**

(56) Entgegenhaltungen:
**DE - B - 1 957 394**

(73) Patentinhaber: **Bayer AG**
**Zentralbereich Patente, Marken und Lizenzen**
**D - 5090 Leverkusen 1, Bayerwerk (DE)**

(72) Erfinder: **Bock, Manfred, Dr.**
**Haydnstrasse 18/D - 5090 Leverkusen 1 (DE)**
**Pedain, Josef, Dr.**
**Haferkamp 6**
**D - 5000 Köln 80 (DE)**
**Slawyk, Wilhelm, Dr.**
**Wolfskaul 12**
**D - 5000 Köln 80 (DE)**
**König, Klaus, Dr.**
**Heymannstrasse 50**
**D - 5090 Leverkusen 1 (DE)**

**EP 0 000 016 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von allophanatgruppen aufweisenden Polyisocyanaten, und ihre Verwendung als Aufbaukomponente für Polyurethankunstoffe

Die vorliegende Erfindung betrifft ein neues Verfahren zur Herstellung von neuen Allophanatgruppen aufweisenden organischen Polyisocyanaten mit mindestens drei Isocyanatgruppen und ihre Verwendung als Aufbaukomponente bei der Herstellung von Polyurethankunststoffen.

Allophanatgruppen aufweisende organische Polyisocyanate mit aliphatische gebundenen Isocyanatgruppen sind beispielsweise aus der GB—PS 994 890 bekannt geworden. Gemäß dieser Patentschrift werden Urethangruppen aufweisende Polyisocyanate aus einfachen ein- oder mehrwertigen Alkoholen und organischen Polyisocyanaten insbesondere Diisocyanaten durch mehrstündiges Erhitzen auf erhöhte Temperaturen bzw. in Gegenwart von Katalysatoren mit weiteren Mengen an organischen Polyisocyanaten vorzugsweise Diisocyanaten zu den Allophanatgruppen aufweisenden Polyisocyanaten umgesetzt. Nachteilhaft an diesem Verfahren ist neben der Mitverwendung von Katalysatoren insbesondere die lange Erhitzungsdauer während der Allophanatisierungsreaktion, welche im allgemeinen zu verfärbten Reaktionsprodukten führt.

Uberraschenderweise wurde nun ein neues Verfahren gefunden, welches die Herstellung von neuen Allophanatgruppen aufweisenden organischen Polyisocyanaten mit aliphatisch bzw. cycloaliphatische gebundenen Isocyanatgruppen gestattet, ohne daß heirfür Katalysatoren oder ein dem Verfahren der GB—PS 994 890 vergleichbares langes Erhitzen erforderlich wäre. Die erfindungsgemäßen Verfahrensprodukte zeichnen sich demzufolge auch durch eine niedrige Farbzahl und darüber hinaus durch eine vergleichsweise niedrige Viskosität aus und stellen wertvolle Ausgangsmaterialien zur Herstellung von Polyurethankunststoffen insbesondere Polyurethanlacken dar.

Die beim nachstehend näher beschriebenen erfindungsgemäßen Verfahren einzusetzenden, speziellen Hydroxylgruppen aufweisenden Verbindungen sind teilweise bereits aus DE—AS 1 957 394 bekannt, wo den beim erfindungsgemäßen Verfahren einzusetzenden Verbindungen entsprechende difunktionelle Hydroxylverbindungen als Kettenverlängerungsmittel empfohlen werden. Die Verwendung von bestimmten Dihydroxyverbindungen als Kettenverlängerungsmittel bei der Herstellung von Polyurethanen ist allerdings in keinerlei näheren Zusammenhang mit der Herstellung von Allophanatgruppen aufweisenden Polyisocyanaten, d.h. von niedermolekularen Ausgangsmaterialien zur Herstellung von Polyurethanen zu bringen.

Gegenstand der vorliegenden Erfindung ist ein Verfahren zur Herstellung von mindestens drei Isocyanatgruppen aufweisenden Allophanat-Polyisocyanaten durch Umsetzung von Hydroxylgruppen aufweisenden Verbindungen mit überschüssigen Mengen an organischen, Allophanatgruppen-freien Polyisocyanaten, dadurch gekennzeichnet, daß man als Hydroxylgruppen aufweisende Verbindung solche der Formel

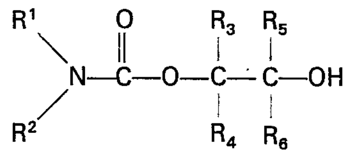

einsetzt, wobei

$R^1$ und $R^2$ für gleiche oder verschiedene Reste stehen und Wasserstoff, Hydroxyalkyl-, Alkyl- oder Cycloalkyl-Reste bedeuten mit der Einschränkung, daß mindestens einer der Reste $R^1$ und $R^2$ für Wasserstoff oder eine Hydroxyalkylgruppe steht, und

$R_3$, $R_4$, $R_5$ und $R_6$ für gleiche oder verschiedene Reste stehen und Wasserstoff, Alkyl- oder Hydroxyalkyl-Reste bedeuten und wobei die Reste $R_3$ und $R_5$ zusammen mit den beiden Kohlenstoffatomen des Grundgerüstes auch einen cycloaliphatischen Ring bilden können.

Gegenstand der vorliegenden Erfindung ist auch die Verwendung der nach diesem Verfahren hergestellten Polyisocyanate als Aufbaukomponente bei der Herstellung von Polyurethankunstoffen nach dem isocyanat-Polyadditionsverfahren.

Bei den erfindungswesentlichen, beim erfindungsgemäßen Verfahren einzusetzenden Hydroxylgruppen aufweisenden Verbindungen handelt es sich vorzugsweise um solche der genannten Formel in welcher.

$R^1$ und $R^2$ für gleiche oder verschiedene Reste stehen und Wasserstoff, $C_2$—$C_{18}$—, insbesondere $C_2$—$C_6$-Hydroxyalkylgruppen, $C_1$—$C_{18}$— insbesondere $C_1$—$C_4$-Alkylgruppen oder $C_4$—$C_{15}$— insbesondere $C_6$—$C_{10}$-Cycloalkylgruppen bedeuten, wobei mindestens einer der Reste $R^1$ oder $R^2$ für Wasserstoff oder eine Hydroxyalkylgruppe steht,

$R_3$, $R_4$, $R_5$ und $R_6$ für gleiche oder verschiedene Reste stehen und Wasserstoff, $C_1$—$C_{13}$— insbesondere $C_1$—$C_2$-Alkylgruppen oder $C_1$—$C_{18}$- insbesondere $C_1$—$C_2$-Hydroxyalkylgruppen bedeuten, wobei die Reste $R_3$ und $R_5$ zusammen mit den beiden Kohlenstoffatomen des Grundgerüsts auch einen cycloaliphatischen Ring mit 5—6 Kohlenstoffatomen darstellen können, und wobei mindestens zwei der Reste $R_3$, $R_4$, $R_5$ und $R_6$ für Wasserstoff stehen.

Ganz besonders bevorzugt sind solche Hydroxylgruppen aufweisende Verbindungen der genannten allgemeinen Formel, für welche $R^1$ und $R^2$ die genannte Bedeutung haben und für welche drei der Reste $R_3$, $R_4$, $R_5$ und $R_6$ für Wasserstoff und einer der genannten Reste für

einen Methyl- oder Hydroxymethylgruppe steht.

Die erfindungswesentlichen Hydroxylgruppen aufweisenden Verbindungen der genannten allgemeinen Formel können leicht durch Umsetzung von Aminogruppen aufweisenden Verbindungen der Formel

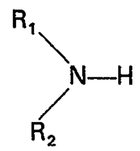

mit cyclischen Carbonaten der Formel

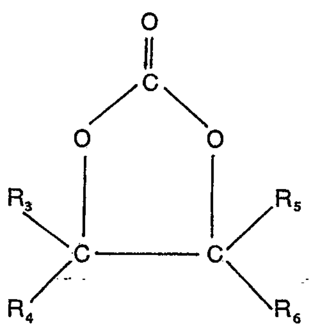

erhalten werden.

In diesen letztgenannten beiden Formeln haben $R_1$ bis $R_6$ die obengenannte Bedeutung.

Beispiele geeigneter Aminogruppen aufweisender Verbindungen sind Ammoniak, Methylamin, Äthylamin, Propylamin, Isopropylamin, Butylamin, sec.-Butylamin, Isobutylamin, tert.-Butylamin, Pentylamin, tert-Pentylamin, Hexylamin, 2-Äthylhexylamin, Dodecylamin, Tetradecylamin, Hexadecylamin, Octadecylamin, 2-Propenylamin, Cyclohexylamin, 2(oder 3 , oder4) - Methylcyclohexylamin, Aminomethylcyclohexylamin, 3,3,5 - Trimethylcyclohexylamin, 2 - Norbornylmethylamin, 2 - Aminoäthanol, 3 - Amino- 1 - propanol, 1 - Amino - 2 - propanol, 4 - Amino - 2 - butanol, 3 - Amino - 1 - butanol, 2 - Amino - 1 - butanol, 3 - Amino - 3 - methyl - 1 - butanol, 2 - Amino - 2 - methyl - 1 - propanol, 2 - Amino - 2 - methyl - 1,3 - propandiol, 2 - Amino - 2 - hydroxymethyl - 1,3 - propandiol, 5 - Amino - 1 - pentanol, 3 - Amino - 2,2,4 - trimethyl - 1 - pentanol, 6 - Amino - 1 - hexanol, Methyl-hexanolamin (Isomerengemisch), Trimethyl - 1,6 - hexanolamin (Isomerengemisch), 2,2 - Dimethyl - 3 - amino - 1 - hexanol, 7 - Amino - 1 - heptanol, 10 - Amino - 1 - decanol, 12 - Amino - 1 - dodecanol, 2(3 oder 4) - Aminocyclohexanol, 2(oder 3) - Methyl - 4 - aminocyclohexanol, 2 - (oder 6) - Methyl - 3 - amino - cyclohexanol, 5(oder 6) - Methyl - 2 - amino - cyclohexanol, 2(3 oder 4) - Aminomethyl - cyclohexanol, 2 - (3 - aminopropyl) - cyclohexanol, 3 - Aminomethyl - 3,3,5 - trimethyl - cyclohexanol, 4 - (2 - Aminoäthyl) - (2 - hydroxyäthyl) - cyclohexan, 1 - Hydroxymethyl - 3(oder 4) - aminomethyl - cyclohexan, 2 - Hydroxymethyl - 5(oder 6) - aminomethyl - bicyclo - 2,2,1 - heptan, 1 - Hydroxy - 5(6 oder 7) - amino - decahydronaphthalon, 2 - Hydroxy - 6(oder 7) - amino - decahydronaphthalin, 1 - Aminomethyl - 2 - hydroxydecahydronaphthalin, 4 - Amino - 4′ - hydroxy - - -dicyclohexylmethan, 2 - (4 - Aminocyclohexyl) - 2 - (4 - hydroxycyclohexyl) - propan, 2 - Methylaminoäthanol, N - (2 - Hydroxyäthyl) - cyclohexylamin, 1 - Cyclohexylamino - 2 - propanol, Bis - (2 - hydroxyäthyl) - amin, Bis - (2 - hydroxypropyl) - amin.

Weiterhin eignen sich aminogruppenhaltige Verbindungen mit Ätherstrukturen, so

3 - Methoxypropylamin, 3 - Äthoxypropylamin, (3 - Aminopropyl) - butyläther oder 4 - Hydroxy - 4′ - Amino - dicyclohexyläther oder solche, die beispielsweise durch Monoaddition von Acrylnitril an Glycole und anschließende Reduktion gewonnen werden können, wie

$$HO—(CH_2)_2—O—(CH_2)_3—NH_2$$

$$HO—CH_2)_4—O—(CH_2)_2—NH_2$$

$$\text{oder } HO—(CH_2)_5—O—(CH_2)_2—NH_2$$

Beispiele geeigneter cyclischer Carbonate sind 1,3 - Dioxolan - 2 - on, 4 - Methyl - 1,3 - dioxolan - 2 - on, 5 - Methyl - 1,3 - dioxan - 2 - on, 5 - Dimethyl - 1,3 - dioxan - 2 - on, 5 - Äthyl - 1,3 - dioxan - 2 - on, 5 - Diäthyl - 1,3 - dioxan - 2 - on, 4 - Hydroxymethyl - 1,3 - dioxolan - 2 - on und 2,4 - Dioxa - bicyclo - 4,3,0 - nonan - 3 - on.

Die Herstellung der erfindungswesentlichen Hydroxylgruppen aufweisenden Verbindungen erfolgt im allgemeinen bei 20 bis 100°C, vorzugsweise 30 bis 50°C durch Umsetzung äquimolarer Mengen der beispielhaft genannten Aminogruppen-haltigen Verbindungen mit den beispiel-haft genannten cyclischen Carbonaten.

Für das erfindungsgemäße Verfahren sind beliebege organische Polyisocyanate insbesondere Diisocyanate mit aliphatisch und/oder cycloaliphatisch gebundenen Isocyanatgruppen geeignet. Beispiele hierfür sind Äthylenediisocyanat, 1,4 - Tetramethylendiisocyanat, 2,2,4 - (2,4,4) - Trimethylhexymethylendiisocyanat - 1,6, 1,12 - Dodecandiisocyanat, Lysin - diisocyanat - $C_1$—$C_8$ - alkylester, Cyclobutan - 1,3 - diisocyanat, Cyclohexan - 1,3- und -1,4 - diisocyanat sowie beliebige Gemische dieser Isomeren, 2,4- und 2,6 - Hexahydrotoluylendiisocyanat sowie beliebige Gemische dieser Isomeren, 3,3′ - Dimethyl - 4,4′ - diisocyanatodicyclohexylmethan, 4,4′ - Diisocyanatocicyclohexylmethan und Xylylendiisocyanat.

Bevorzugt werden Hexamethylendiisocyanat- und 1 - Isocyanato - 3,3,5 - tri-

methyl - 5 - isocyanatomethyl - cyclohexan (Isophorondiisocyanat) eingesetzt.

Das erfindungsgemäße Verfahren wird im Temperaturbereich von 90—200°C, vorsugsweise 100—180°C durchgeführt. Bei der Durchführung des erfindungsgemäßen Verfahrens gelangen die Reaktionspartner in solchen Mengenverhältnissen zum Einsatz, die einem NCO/OH - Äquivalentverhältnis von mindestens 4:1, vorzugsweise 6:1 bis 25:1 entsprechen.

Die Durchführung des erfindungsgemäßen Verfahrens kann beispielsweise folgendermaßen erfolgen:

6—25 NCO-Äquivalente eines aliphatischen oder cycloaliphatischen Diisocyanats werden in einem Rührgefaß unter Inertgasatmosphäre (Stickstoff oder Argon) vorgelegt und auf 100—180°C erwärmt. Anschließend trägt man ein Hydroxyläquivalent der erfindungwesentlichen Hydroxylverbindung unter intensivem Rühren in die Isocyanatvorlage ein. Danach erhitzt man noch 20 Minuten bis etwa 4 Stunden auf 100—180°C. In dieser Zeit stellt sich ein konstanter NCO-Gehalt ein. Die Reaktion ist hiernach beendet und das überschüssige Isocyanat wird nach einem bekannten Destillations- oder Extraktionsverfahren (beispielsweise unter Verwendung von n-Hexan oder Cyclohexan) abgetrennt.

Das erfindungsgemäße Verfahren läßt sich in gleicher Weise gut auch zweistufig durchführen, wobei die Ausgangskomponenten bei Raumtemperatur zusammengerührt werden und anschließend gemeinsam auf den obengenannten Temperaturberiech von 100—180°C erhitzt werden. Auch die Mitverwendung eines gegenüber Isocyanaten inerten Lösungsmittels wie z.B. Äthylacetat, Butylacetat, Toluol oder Xylol ist möglich. Vorzugsweise wird das erfindungsgemäße Verfahren jedoch lösungsmittelfrei durchgeführt.

Die Verfahrensprodukte sind viskose, farblose bis gelb gefärbte Polyisocyanate, die bei Raumtemperatur flüssig sind oder als feste Hartharze vorliegen. Sie sind völlig geruchlos und klar in genenüber NCO-Gruppen inerten Lösungsmitteln wei Kohlenwasserstoffen, Chlorkohlenwasserstoffen, Estern und Ketonen löslich.

Bei der Durchführung des erfindungsgemäßen Verfahrens entstehen aus den Hydroxylgruppen der erfindungswesentlichen Hydroxylgruppen aufweisenden Verbindung und einem Teil der Isocyanatgruppen des Diisocyanats zunächst Urethangruppen. Diese somit als Zwischenstufe anfallenden Urethangruppen aufweisenden Polyisocyanate bilden sich bereits im Temperaturbereich zwischen 50 und 100°C und können bei entsprechender Temperaturführung isoliert werden, da bei diesen Temperaturen im wesentlichen noch keine Allophanatisierung eintritt. Bei höherer Temperatur, oberhalb von 100°C, addiert sich weiteres Diisocyanat an die Urethangruppen unter Ausbildung von Allophanaten. Im Falle einer Umsetzung des Urethanalkohols mit Diisocyanat bei einer von Beginn an hohen Temperatur von beispielsweise 150°C verlaufen Urethanisierung der OH-Funktion und Allophanatisierung unselektiv und nebeneinander ab.

Die genannte selektive Reaktionsführung durch entsprechende Steuerung der Reaktionstemperaturen eröffnet die interessante Möglichkeit, Allophanatgruppen aufweisende Polyisocyanate einer definierten Struktur aufzubauen, in denen beispielsweise sowohl aliphatisch als auch cycloaliphatisch gebundene Isocyanatgruppen vorliegen. So ist es beispielsweise möglich zunächst im Temperaturbereich zwischen 50 und 100°C unter Verwendung eines NCO/OH-Äquivalentverhältnisses von 1:1 gezielt ein Urethangruppen aufweisendes Polyisocyanat herzustellen, welches entweder ausschließlich aliphatisch oder ausschließlich cycloaliphatisch gebundene Isocyanatgruppen aufweist, um anschließend die Allophanatisierungsreaktion im Temperaturbereich von 100—180°C insbesondere 110—150°C unter Verwendung von überschüssigen Mengen eines cycloaliphatischen bzw. aliphatischen Diisocyanat durchzuführen.

Für diese selektive Reaktionsführung besonders gut geeignete erfindungswesentliche Hydroxylgruppen aufweis-ende Verbindungen sind insbesondere die entsprechenden Additionsprodukte von Ammoniak an die beispielhaft genannten cyclischen Carbonate.

Bei allen Ausführungformen des erfindungsgemäßen Verfahrens kann im Anschluß an die erfin-dungsgemäße Umsetzung das gegebenenfalls noch vorliegende überschüsige Diisocyanat destillativ beispielsweise in Dünnschichtverdampfern abgetrennt werden.

Das erfindungsgemäße Verfahren weist insbesondere folgende Vorteille auf:

1. es ermöglicht die Herstellung von physiologisch weitgehend unbedenklichen Allophanatpolyisocyanaten einer niedrigen Viskosität, die insbesondere in Lösungsmittelfreien bzw.-armen Lacken eingesetzt werden können;

2. die erfindungsgemäß zugänglichen Verfahrensprodukte weisen eine helle Eigenfarbe auf und eignen sich aus diesem Grund und aufgrund der Tatsache, daß die Isocyanatgruppen aliphatisch bzw. cycloaliphatisch gebunden sind besonders gut zur Herstellung von lichtechten Polyurethanlacken und

3. das erfindungsgemäße Verfahren gestattet aufgrund der oben beschriebenen Moglichkeit einer selektiven Reaktionsführung die Herstellung von definierten Polyisocyanaten, deren physikalische und chemische Eigenschaften durch geeignete Wahl der verschiedenen Ausgangsdiisocyanate dem jeweiligen Verwendungszweck angepaßt werden können.

Die erfindungsgemäßen Verfahrensprodukte stellen wertvolle Ausgangsmaterialien zur Herstellung von Polyurethankunststoffen nach

dem Isocyanat-Polyadditionsverfahren, insbesondere zur Herstellung von Ein- oder Zweikomponenten-Polyurethanlacken dar. Bei Verwendung der erfindungsgemäßen Verfahrensprodukte in mit bekannten Blockierungsmitteln für Isocyanatgruppen blockierter Form eignen sich die erfindungsgemäßen Verfahrensprodukte insbesondere auch zur Herstellung von Polyurethan-Einbrennlacken.

Bevorzugte Reaktionspartner für die erfindungsgemäßen, gegebenenfalls in blockierter Form vorliegenden Verfahrensprodukte bei der Herstellung von Polyurethanlacken sind die in der Polyurethanlacktechnik an sich bekannten Polyhydroxypolyester, Polyhydroxypolyacrylate und gegebenenfalls niedermolekularen, mehrwertigen Alkohole. Geeignete derartige Reaktionspartner sind beispielsweise in DT—AS 2 304 893 beschrieben.

Die Mengenverhältnisse, in welchen die erfindungsgemäßen, gegebenenfalls blockierten Polyisocyanate und die genannten Reaktionspartner bei der Herstellung von Polyurethanlacken umgesetzt werden, werden im allgemeinen so gewählt, daß auf eine (gegebenenfalls blockierte) Isocyanatgruppe 0,8—3, vorzugsweise 0,9—1,1, Hydroxyl, Amino-, Mercapto- und/oder Carboxylgruppen entfallen.

Zur Beschleunigung der Aushärtung können in bekannter Weise die in der Isocyanatchemie üblichen Katalysatoren verwendet werden, wie z.B. tert. Amine wie Triäthylamin, Pyridin, Methylpyridin, Benzyldimethylamin, N,N-Dimethylaminocyclohexan, N-Methylpiperidin, Pentamethyldiäthylentriamin, N,N' - Endoäthylenpiperazin, N,N' - Dimethylpiperazin usw., Metallsalze wie Eisen(III) - chlorid, Zinkchlorid, Zink - 2 - äthylcaproat, Zinn(II) - 2 - äthylcaproat, Dibutylzinn(IV) - dilaurat, Molybdänglykolat usw..

Bei Anwendung der Allo-phanat-Polyisocyanate in Einkomponentenlacken finden ebenfalls vor allen die genannten OH-funktionellen Reaktionspartner Verwendung.

Sie werden in einem Mengenverhältnis umgestzt, daß auf eine OH-Gruppe mindestens 1,2, vorzugsweise 1,5 bis 10, NCO-Gruppen entfallen. Bei der Umsetzung entstehen Lackbindemittel mit freien NCO-Gruppen, die an feuchter Luft zu harten, glänzenden und hochwertigen Beschichtungen aushärten. Die genannten Katalysatoren können auch bei Einkomponenten-Lacken eingesetzt werden.

Bei Verwendung der Allophanat-Polyisocyanate in Einbrennlacken werden die NCO-Gruppen ganz oder teilweise in bekannter Weise blockiert. Das Polyisocyanat wird mit einem geeigneten Blockierungsmittel, vorzugsweise bei erhöhter Temperatur (z.B. 40 bis 140°C) gegebenenfalls in Gegenwart eines geeigneten Katalysators, wie z.B. tert. Amine, Metallsalze wie Zink - 2 - äthylcaproat, Zinn(II) - 2 - äthylcaproat, Dibutylzinn(IV) - dilaurat oder Alkaliphenolat umgesetzt.

Geeignete Blockierungsmittel sind beispielsweise:

Monophenole wie Phenol, die Kresole, die Trimethylphenole, die tert. Butylphenole; tertiäre Alkohole wie tert.-Butanol, tert.-Amylalkohol, Dimethylphenylcarbonol; leicht Enole bildende Verbindungen wie Acetessigester, Acetylaceton, Malonsäurederivate wie Malonsäurediester mit 1 bis 8 C-Atomen in den Alkoholresten; sekundäre aromatische Amine wie N - Methylanalin, die N - Methyltoluidine, N - Phenyltoluidin, N - Phenylxylidin; Imide wie Succinimid; Lactame wie $\varepsilon$-Caprolactam, $\delta$-Valerolactam; Oxime wie Butanonoxim, Cyclohexanonoxim; Mercaptane wie Methylmercaptan, Äthylmercaptan, Butylmercaptan, 2 - Mercaptobenzthiazol, $\alpha$-Naphthylmercaptan, Dodecylmercaptan.

Zur Herstellung der Lackbindemittel werden gegebenenfalls blockiertes Polyisocyanat, polyfunktioneller Reaktionspartner, Katalysator und gegebenenfalls die üblichen Zusätze, wie z.B. Pigmente, Farbstoffe, Füllstoffe und Verlaufmittel miteinander auf einem üblichen Mischaggregat, wie z.B. auf einer Sandmühle entweder mit oder ohne Lösungsund Verdünnungsmittel gut vermischt und homogenisiert.

Die Anstrich- und Überzugsmittel können in lösungsmittelfreier flüssiger Form oder in Lösung oder aus der Schmelze, oder in fester Form nach den üblichen Methoden wie z.B. Streichen, Rollen, Gießen, Spritzen, dem Wirbelsinterverfahren oder dem electrostatischen Pulversprühverfahren auf den zu beschichtenden Gegenstand aufgebracht werden.

Die die erfindungsgemäßen Polyisocyanate enthaltenden Lacke ergeben Filme, die überraschend gut auf metallischem Untergrund haften, besonders lichtecht, wärmefarbstabil und sehr ariebfest sind und, sofern sie in lufttrocknenden Lacken verwendet werden, besonders rasch, selbst bei Temperaturen um 0°C antrocknen. Darüber hinaus zeichnen sie sich durch große Härte, Elastizität, sehr gute Chemikalienbeständigkeit, hohen Glanz, ausgezeichnete Wetterbeständigkeit und gute Pigmentierbarkeit aus.

Die folgenden Beispiele erläutern die Erfindung. Alle Prozentangaben betreffen Gewichtsprozente.

In den nachfolgenden Beispielen 2, 4, 5, 6, 8, 10, 11, 12, 14 und 16 werden als Reaktionspartner für die Diisocyanate bei der Durchführung des erfindungsgemaßen Verfahrens Homologengemische eingesetzt, die als solche bein ihrer Herstellung anfallen. So entsteht beispielsweise das in Beispiel 2 verwendete Homologengemisch aus N - (2 - Hydroxyäthyl) - 2 - hydroxy - 1 - methyläthylcarbamat und N - (2 - Hydroxyäthyl) - 2 - hydroxy - 2 - methyläthylcarbamat bei der Umsetzung von 2 - Aminoäthanol und 4 - Methyl - 1,3 - dioxolan - 2 - on. In dem Homologengemisch kann der Methyl-Substituent sowohl in 1- als auch in 2-Stellung vorliegen.

Beispiel 1

2018 g (12 Mol) Hexamethylendiisocyanat werden in einem Dreihalskolben unter Stickstoffatmosphäre vorgelegt und auf 150°C erhitzt. Aus einem Tropftrichter werden hierzu innerhalb von 20 Minuten 149 g (1 Mol) N - (2 - Hydroxyäthyl) - 2 - hydroxyäthylcarbamat zugetropft. Hiernach rührt man 2 Stunden bei 150°C nach. Zur Isolierung des Polyisocyanats wird das Raktionsgemisch im Hochvakuum 0,27 mbar bei 170°C Beheizung gedünnschichtet, d.h. das überschüssige Diisocyanat wird im Dünnschichtverdampfer destillativ entfernt und das Verfahrensprodukt als Destillationsrückstand isoliert.

| | |
|---|---|
| Ausbeute: | 720 g |
| Viskosität: | 40 000 mPa.s/25°C |
| NCO-Gehalt: | 17,5 % |

Beispiel 2

Analog Beispiel 1 werden 2018 g (12 mol) Hexamethylendiisocyanat bei 150°C mit 163 g (1 Mol) N - (2 - Hydroxyäthyl) - 2 - hydroxy - 1 (oder 2) - methyläthylcarbamat innerhalb von 20 Minuten versetzt. Nach 2 - stünden Nachrühren wird abgekühlt und gedünnschichtet (170°C/0,13 mbar).

| | |
|---|---|
| Ausbeute Polyisocyanat | 680 g |
| Viskosität | 18 000 mPa.s |
| NCO-Gehalt: | 17,4 % |

Beispiel 3

In die auf 150°C erhitzte Vorlage von 2018 g (12 Mol) Hexamethylendiisocyanat werner innerhalb von ca. 20 Minuten 163 g (1 Mol) N - (3 - Hydroxypropyl) - 2 - hydroxyäthylcarbamat eingetropft. Nach 4 - stündigem Nachrühren bei 150°C ist die Reaktion beendet. Nach Dünnschichtdestillation wird das Polyisocyanat als Destillationsrückstand isoliert.

| | |
|---|---|
| Ausbeute: | 700 g |
| Viskosität: | 18 300 mPa.s/25°C |
| NCO-Gehalt: | 21,7 % |

Beispiel 4

2018 g (12 Mol) Hexamethlendiisocyanat werden vorgelegt und auf 150°C erhitzt. Hierzu tropft man 177 g (1 Mol) N - (3 - Hydroxypropyl) - 2 - hydroxy - 1(oder - 2) - methyl - äthylcarbamat innerhalb von ca. 20 Minuten. Nach zweistündigem Nachrühren ist die Reaktion beendet. Das Polyisocyanat wird durch Dünnschichtdestillation als Destillationsrückstand isoliert.

| | |
|---|---|
| Ausbeute: | 600 g |
| Viskosität: | 5 500 mPa.s/25°C |
| NCO-Gehalt: | 17,4 % |

Beispiel 5

2018 g (12 Mol) Hexamethylendiisocyanat werden bei 150°C innerhalb von ca. 20 Minuten mit 177 g (1 Mol) N - (2 - Hydroxypropyl) - 2 - hydroxy - 1(oder - 2) - methyl - äthylcarbamat versetzt. Nach einstündigem Nachrühren wird das Polyisocyanat durch Dünnshichtdestillation als Destillationsrückstand isoliert.

| | |
|---|---|
| Ausbeute: | 650 g |
| Viskosität: | 12 000 mPa.s/25°C |
| NCO-Gehalt: | 18,0 % |

Beispiel 6

1009 g (6 Mol) Hexamethylendiisocyanat werden vorgelegt und auf 150°C erwärmt. Innerhalb von ca. 15 Minuten werden 95 g (0,5 Mol) N - (1,1' - Dimethyl - 2 - hydroxyäthyl) - 2 - hydroxy - 1(odar - 2) - methyl - äthylcarbamat in die Vorlage eingetropft. Nach 30-minütigem Nachrühren wird da Reaktionsgemisch gedünnschichtet.

| | |
|---|---|
| Ausbeute: | 350 g |
| Viskosität: | 4 200 mPa.s/25°C |
| NCO-Gehalt: | 17,2 % |

Beispiel 7

2018 g (12 Mol) Hexamethylendiisocyanat werden in einem Dreihalskolben auf 150°C erhitzt und bei dieser Temperatur innerhalb von ca. 20 Minuten mit 105 g (1 Mol) 2 - Hydroxyäthylcarbamat versetzt. Nach 4-stündigem Nachrühren wird das Reaktionsgemisch gedünnschichtet und so das Polyisocyanat als Destaillsationsrückstand isoliert.

| | |
|---|---|
| Ausbeute: | 660 g |
| Viskosität: | 27 000 mPa.s/25°C |
| NCO-Gehalt: | 17,5 % |

Beispiel 8

In die auf 150°C erhitzte Vorlage von 2018 g (12 Mol) Hexamethylendiisocyanat werden innerhalb von ca. 20 Minuten 119 g (1 Mol) 2 - Hydroxy - 1 - (oder - 2) - methyläthylcarbamat eindosiert. Nach dreistündigem Nachrühren ist die Reaktion beendet, das Reaktionsprodukt wird am Dünnschichtverdampfer destilliert und das Reaktionsprodukt als Destillationsrückstand isoliert.

Ausbeute: 909 g

Viskosität: 37 000 mPa.s/25°C

NCO-Gehalt: 18,9 %

Beispiel 9

1009 g (6 Mol) Hexamethylendiisocyanat werden zusammen mit 80 g (0,5 Mol) 2 - Hydroxycyclohexylcarbamat vorgelegt und auf 150°C erhitzt. Zwischen 100 und 110°C schmilzt das Carbamat im Diisocyanat und geht allmählich in Reaktion. Nach zweistündigem Nachrühren bei 150°C wird der Versuch beendet und das Umsetzungsprodukt gedünnschichtet.

Ausbeute: 386 g

Viskosität (75 %ig in Äthylglykolacetat): 8 500 mPa.s/25°C

NCO-Gehalt (75 %ige Lösung): 12,1 %

Beispiel 10

90 g (0,5 Mol) N - (propyl) - 1 - (oder - 2) - hydroxymethyl - 2 - hydroxyäthyl - carbamat werden innerhalb von 15 Minuten in eine Vorlage von 1009 g (6 Mol) Hexamethylendiisocyanat von 150°C eingetropft. Nach 2 Stunden ist die Reaktion beendet. Das Umsetzungsprodukt wird im Hochvakuum gedünnschichtet.

Ausbeute: 343 g

Viskosität: 10 000 mPa.s/25°C

NCO-Gehalt: 18,4 %

Beispiel 11

108 g (0,5 Mol) N - (Cyclohexyl) - 1(oder - 2) - hydroxymethyl - 2 - hydroxy - äthylcarbamat werden innerhalb von 10 Minuten in 1009 g (6 Mol) Hexamethylendiisocyanat von 150°C eindosiert. Nach vierstündigem Nachrühren wird das Reaktionsprodukt gedünnschichtet und das als Destillationsrückstand erhaltene Polyisocyanat 75 %ig in Äthylglykolacetat gelöst.

Ausbeute (100 %): 350 g

Viskosität (Lösung): 390 mPa.s/25°C

NCO-Gehalt (Lösung): 13 %

Beispiel 12

1009 g (6 Mol) Hexamethylendiisocyanat werden vorgelegt und auf 150°C erhitzt. Hierzu tropft man innerhalb von 10 Minuten 103 g (0,5 Mol) N - (bis - (2 - hydroxyäthyl)) - 2 - hydroxy - 1 - (oder - 2) - methyläthylcarbamat. Nach 4 Stunden 150°C ist die Reaktion beendet, das Umsetzungsprodukt wird gedünnschichtet.

Ausbeute: 400 g

Viskosität: 1 500 mPa.s/25°C

NCO-Gehalt: 17,5 %

Beispiel 13

504 g (3 Mol) Hexamethylendiisocyanat werden vorgelet und bei 150°C innerhalb von 5 Minuten mit 48 g (0,25 Mol) N - (bis - (2 - Hydroxyäthyl)) - 2 - hydroxyäthylcarbamat aus einem Tropftrichter versetzt. Man läßt 4 Stunden bei 150°C nachrühren, filtriert das Reaktionsprodukt von einer eventuell vorhandenen Trübung ab und dünnschichtet im Hochvakuum.

Ausbeute: 182 g

Viskosität: 20 000 mPa.s/25°C

NCO-Gehalt: 18,1 %

Beispiel 14

52 g (0,25 Mol) N - (1,1' - Dihydroxymethyl - äthyl) - 2 - hydroxy - 1 (oder 2) - methyl-äthylcarbamat werden zusammen mit 1009g (6 Mol) Hexamethylendiisocyanat 2 1/2 Stunden auf 150°C erhitzt. Hiernach wird das überschüssige Hexamethylendiisocyanat im Dünnschichverdampfer abgetrennt.

Ausbeute: 233 g

Viskosität: 12 000 mPa.s/25°C

NCO-Gehalt: 17,8 %

In den folgenden Beispielen (15, 16) wird die Darstellung von Polyisocyanaten beschrieben, die sowohl cycloaliphatisch als auch linear gebunden endständige NCO-Gruppen aufweisen.

Beispiel 15

111 g (1/2 Mol) Isophorondiisocyanat werden vorgelegt und bei 80°C tropfenweise mit 53 g (1/2 Mol) 2-Hydroxyäthylcarbamat versetzt. Die Umsetzung bis zum theoretischen NCO-Gehalt erfolgt bei 80°C innerhalb von ca. 4 Stunden. Anschließend läßt man in das Reaktionsgemisch 1009 g (6 Mol) Hexamethylendiisocyanat einfließen und erhöht die Temperatur auf 150°C. Nach 3 Stunden ist die Reaktion beendet und man dünnschichtet das Umsetzungsprodukt in Hochvakuum.

Ausbeute (100 %): 315 g

Viskosität (80 %ig in Äthylglykoacetat:

NCO-Gehalt (80 %ige Lösung): 13,5 %

Beispiel 16

120 g (1 Mol) 2 - Hydroxy - 1(oder - 2) - methyläthylcarbamat werden bei 80°C in 222 g (1 Mol) vorgelegtes Isophorodiisocyanat eingetropft. Nach 4-Stündigem Nachrühren bis zum Erreichen des berechneten NCO-Gehaltes, wird das Reaktionsgemisch mit 2018 g (12 Mol) Hexamethylendiisocyanat versetzt und 4 Stunden auf 150°C erhitzt. Hiernach wird das Umsetzungsprodukt durch Abdünnschichten des überschüssigen Isocyanats isoliert und 80 %ig in Äthylglykolacetat gelöst.

| | |
|---|---|
| Ausbeute: | 630 g (100 %) |
| Viskosität: | 6 100 mPa.s/25°C (80 %) |
| NCO-Gehalt: | 12,8 % (80 %) |

Beispiel 17

Die Prüfung auf Stabilität der dargestellten Polyisocyanate erfolgt an einigen Beispielen durch mehrwöchiges Tempern bei 50°C und anschließender gaschromatographischer Analyse der Proben auf ihren Gehalt an monomerem Isocyanat.

Gehalt an monomerem (%) Diisocyanat

| Polyisocyanat aus Beispiel | O-Wert | nach 4 Wochen | nach 8 Wochen | nach 12 Wochen | nach 16 Wochen |
|---|---|---|---|---|---|
| 2 | 0,72 | 0,74 | 0,74 | 0,7 | 0,73 |
| 4 | 0,54 | 0,31 | 0,23 | 0,45 | 0,48 |
| 5 | 0,28 | 0,26 | 0,29 | 0,27 | — |
| 6 | 0,18 | 0,21 | 0,20 | 0,24 | — |
| 8 | 0,79 | 0,72 | 0,75 | — | — |
| 16 | 0,64 | 0,63 | 0,65 | — | — |

Dieser Versuch zeigt besonders anschaulich, daß die erfindungsgemäßen Verfahrensprodukte gegen Monomerenrückspaltung weitgehend stabil sind, was in physiologischer Hinsicht ein besonders wichtiges Kriterium für ihre Eignung als Lack-Polyisocyanat darstellt.

**Patentansprüche**

1. Verfahren zur Herstellung von mindestens drei Isocyanatgruppen aufweisenden, Allophanat-Polyisocyanaten durch Umsetzung von Hydroxylgruppen aufweisenden Verbindungen mit überschüssigen Mengen an organischen, Allophanatgruppen-freien Polyisocyanaten, dadurch gekennzeichnet, daß man als Hydroxylgruppen aufweisende Verbindungen solche der Formel

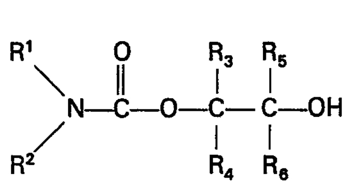

einsetzt, wobei

$R^1$ und $R^2$ für gleiche oder verschiedene Reste stehen und Wasserstoff, Hydroxyalkyl-, Alkyl- oder Cyclo-alkyl-Reste bedueten mit der Einschränkung, daß mindestens einer der Reste $R^1$ und $R^2$ für Wasserstoff oder eine Hydroxyalkylgruppe steht, und

$R_3$, $R_4$, $R_5$ und $R_6$ für gleiche oder verschiedene Reste stehen und Wasserstoff, Alkyl- oder Hydroxyalkyl-Reste bedeuten und wobei die Reste $R_3$ und $R_5$ zusammen mit den beiden Kohlenstoffatomen des Grundgerüstes auch einen cycloaliphatischen Ring bilden können.

2. Verwendung der gemäß Anspruch 1 hergestellten Allophanatgruppen aufweisenden Polyisocyanate gegebenenfalls in mit Blockierungsmitteln für Isocyanatgruppen blockierter Form als Isocyanat-Komponente bei der Herstellung von Polyurethankunststoffen nach dem isocyanat-Polyadditionsverfahren.

**Claims**

1. A process for the preparation of allophanate polyisocyanates having at least three isocyanate groups by the reaction of hydroxyl compounds with excess quantities of organic polyisocyanates which are free from allophanate groups, characterized in that the hydroxyl compounds used are represented by the formula

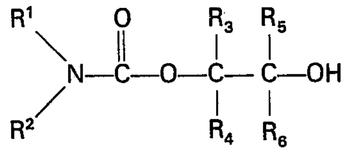

wherein

$R^1$ and $R^2$ are identical or different and represent hydrogen or hydroxyalkyl, alkyl or cycloalkyl groups but at least one of the groups $R^1$ and $R^2$ must be hydrogen or a hydroxy alkyl group, and

$R_3$, $R_4$, $R_5$ and $R_6$ are identical or different and represent hydrogen or alkyl or hydroxy alkyl groups or the groups $R_3$ and $R_5$ together with the two carbon atoms may form the basic structure of a cycloaliphatic ring.

2. Use of the allophanate-polyisocyanates obtained according to Claim 1, optionally in the form of polyisocyanates blocked with blocking agents for isocyanate groups, as isocyanate components for the production of polyurethane resins by the isocyanate polyaddition process.

**Revendications**

1. Procédé de production de polyisocyanates porteurs de groupes allophanate, présentant au moins trois groupes isocyanate, par réaction de composés porteurs de groupes hydroxyle avec des quantités en excès de polyisocyanates organiques dépourvus de groupes allophanate, caractérisé en ce qu'il consiste à utiliser comme composés portant des groupes hydroxyle, des composés de formula:

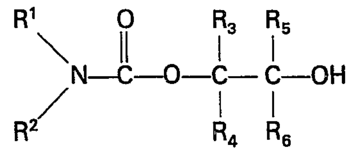

dans laquelle

$R^1$ et $R^2$ sont des restes égaux ou différents et représentent de l'hydrogéne ou des restes hydroxyalkyle, alkyle ou cycloalkyle, à condition qu'au moins l'un des restes $R^1$ et $R^2$ soit un atome d'hydrogène ou un groupe hydroxyalkyle et

$R_3$, $R_4$, $R_5$ et $R_6$ sont des restes égaux ou différents et représentent de l'hydrogène ou des restes alkyle ou hydroxyalkyle, les restes $R_3$ et $R_5$ pouvant former également un noyau cycloaliphatique avec les deux atomes de carbone du squelette de base.

2. Utilisation des polyisocyanates porteurs de groupes allophanate, obtenus conformément à la revendication, 1, le cas échéant sous la forme bloquée par des agents de blocage des groupes isocyanate, comme composant isocyanate dans la production de matières plastiques du type polyuréthanne par le procédé de polyaddition d'isocyanates.